# EUROPEAN PATENT SPECIFICATION

(11) **EP 1 044 976 B1**
(45) Date of publication and mention of the grant of the patent: **24.07.2002**
(21) Application number: 00107836.9
(22) Date of filing: 12.04.2000
(51) Int. Cl.: C07D 487/06, A61K 31/495

(54) **Process for the preparation of pirlindole hydrochloride**
Verfahren zur Herstelung von Pirlindolehydrochlorid
Procédé de préparation de chlorhydrate de pirlindole

(30) Priority: 16.04.1999 IT MI990802
(43) Date of publication of application: 18.10.2000
(73) Proprietor: ERREGIERRE S.p.A., 24060 San Paolo D'Argon (Bergamo) (IT)
(72) Inventor: Ferrari, Massimo, 24069 Cenate Sotto (Bergamo) (IT); Ghezzi, Marcello, 24035 Curno (Bergamo) (IT); Belotti, Paola, 24060 San Paolo D'Argon (Bergamo) (IT); Zinetti, Fabrizio, 24060 Casazza (Bergamo) (IT)
(74) Representative: Gervasi, Gemma, Dr.

(56) References cited:
- CHEMICAL ABSTRACTS, vol. 107, no. 21, 1987 Columbus, Ohio, US; abstract no. 198249g, IVANOV ET AL.: "A new approach to the synthesis of pyrazidol" page 757; XP002121935 & KHIM.-FARM. ZH., vol. 21, no. 1, 1987, pages 71-5,

## Description

### Scope of invention

The present invention refers to an improved process for the preparation of pirlindole hydrochloride.

### State of the art

Pirlindole (1,10-trimethylene-8-methyl-1,2,3,4-tetrahydropyrazine[1,2-a]-indol) hydrochloride of formula (I) 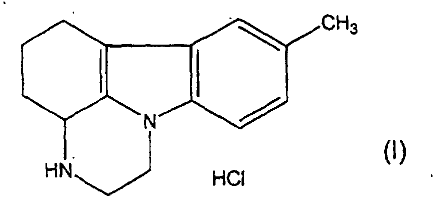 is an antidepressant that acts both on the recovery of noradrenaline and as a MAO (monoamine-oxidase) inhibitor ("Drugs of the future", Vol. V, No.1, pp. 39-41, 1980).

Various processes have been described for the synthesis of this molecule, and the majority of these starts from 6-methyl-1,2,3,4-tetrahydrocarbazol-1-one of formula (II)

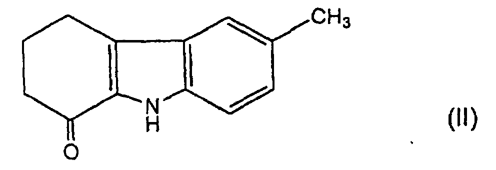

For example the patent FR 2,132,514 (in the name of Vsesojuzny Nauchno-Issledovatelsky Khimiko-Farmatsevtichesky Institut Imeni Sergo Ordzhonikidze) illustrates a process starting right from the compound of formula (II) which, when made to react with an alkaline agent such as sodium methoxide or ethoxide, and, subsequently, with chloro-acetonitrile, yields 9-cyanomethyl-6-methyl-1,2,3,4-tetrahydrocarbazol-1-one which, by catalytic hydrogenation, preferably with nickel Raney or Adams' catalyst (platinum oxide), and subsequent precipitation from an alcoholic or etheric solution of hydrochloric acid, leads to the desired product. It is clear that the said process, both for reasons of safety (chloro-acetonitrile is an extremely toxic substance; nickel Raney is a readily explosive compound) and for economic reasons (Adams' catalyst is notably costly), is not easy to apply at an industrial level.

Ivanov, P. Yu. *et al.,* Khim.-Farm. Zh. (1987), 21(1), pp. 71-75 (hereinafter "Ivanov *et al*.") describes a process that does not present these drawbacks. Also this starts from 6-methyl-1,2,3,4-tetrahydrocarbazol-1-one of formula (II) which, when made to react with ethanol amine, yields the 6-methyl-1-(2-hydroxy-imino)-1,2,3,4-tetrahydrocarbazol of formula (III)

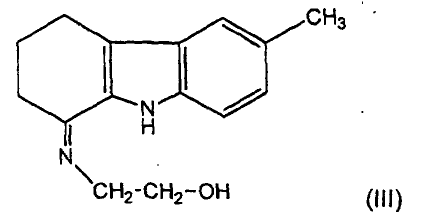

This is first salified with hydrochloric acid in ethanol solution, then treated with thionyl chloride to yield the 6-methyl-1-(2-chloroethyl-imino)-1,2,3,4-tetrahydrocarbazol hydrochloride of formula (IV) 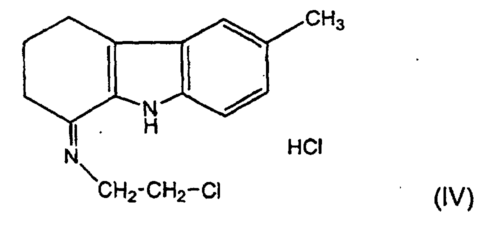 which by reduction with sodium borohydride yields the 6-methyl-1-(2-chloroethylamino)-1,2,3,4-tetrahydrocarbazol hydrochloride of formula (V)

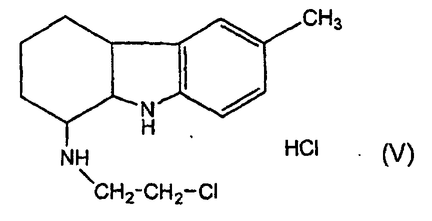

This intermediate is subjected to an intramolecular cyclization reaction to yield the desired compound. The said cyclization is conducted by means of an alkaline agent in aqueous solution, for example sodium hydroxide, in the presence of a phase-transfer catalyst, using benzene as reaction solvent.

The alkaline agent is used in great excess: the example given by this article reports the use of a 50% aqueous solution obtained starting from 15 g of sodium hydroxide (2.8 mol.) as compared to 9.07 g of the derivative of formula (V) (30 mmol.).

The same example gives a quantity of phase-transfer catalyst of 10 wt% with respect to the same derivative of formula (V).

The final-product yield of this process is 68.4% calculated with respect to the intermediate of formula (IV).

### Summary of the invention

We have now found a method of synthesis of pirlindole hydrochloride that not only represents an industrially applicable alternative to the processes of the known art, but also presents advantages with respect to the method proposed by Ivanov *et al.* in that it uses smaller quantities of reagents whilst still obtaining the same yield.

### Description of the invention

Consequently, the present invention regards a process for the synthesis of pirlindole hydrochloride of formula (I) 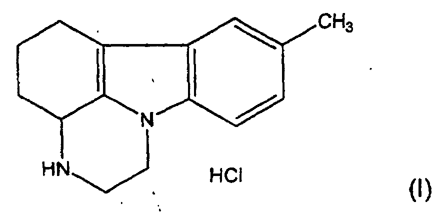 characterized in that the 6-methyl-1-(2-chloroethyl-imino)-1,2,3,4-tetrahydrocarbazol hydrochloride of formula (IV) 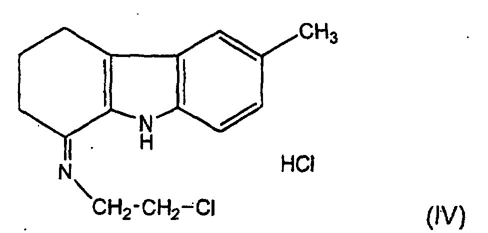 cyclizes to yield the 1,2,5,6-tetrahydro-8-methyl-pyrazine[3,2,1-j,k]-4H-carbazol of formula (VI) 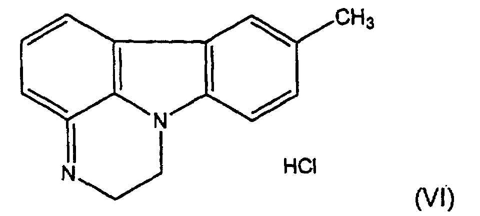 which is subjected to reduction.

More specifically the present invention regards a process in which the compound of formula (IV) is cyclized by treatment with an alkaline agent in a molar ratio of from 1.5:1 to 3:1, in the presence of a phase-transfer catalyst at 3 wt%, to yield the compound of formula (VI), which is then reduced to yield the compound of formula (I).

The alkaline agent is a carbonate of an alkaline metal, such as sodium or potassium carbonate, preferably potassium carbonate.

Preferably the molar ratio between the alkaline agent and the compound of formula (IV) is 2.4:1.1.

The phase-transfer catalyst is chosen from the group comprising tetrabutyl ammonium bromide and tetrabutyl ammonium hydrogen phosphate. For purely practical reasons, the use of tetrabutyl ammonium bromide is preferred.

The cyclization reaction occurs at a temperature of between 80°C and 140°C, preferably at 120°C, for a period of approximately 3 hours.

The reduction reaction occurs at a temperature of between 80°C and 120°C, preferably at 100°C, for a period of approximately 5 hours.

The compound of formula (VI) is reduced with sodium borohydride.

The entire process of the invention is conducted in dimethyl formamide, dimethyl acetamide or ethylene glycol monomethyl ether, dimethyl formamide being preferred.

The yield of the process that forms the subject of the present invention is equivalent to that of Ivanov *et al.,* namely, around 70% with respect to the compound of formula (IV). The final product is obtained with a degree of purity higher than 99%.

As already above said, the process of the present invention presents the advantage of using very reduced quantities of both alkaline agent and phase-transfer catalyst in the intramolecular cyclization reaction, even though the same yield in final product is obtained. More specifically, the quantity of phase-transfer catalyst is more than halved with respect to the amount used by Ivanov *et al.,* whilst the quantity of alkaline agent is at least three times lower than that of the same process.

The fact of diminishing the quantity of reagents does not represent an advantage only from the economic point of view, but also as regards the operating conditions, which are rendered more convenient by the decrease in the reaction volumes.

A further advantage of the present process with respect to that of Ivanov *et al.* lies in the fact of having replaced benzene, a solvent that is notoriously carcinogenic and hence problematical to use above all in the pharmaceutical field, with dimethyl formamide or one of the other solvents listed above. The transition from benzene to dimethyl formamide, dimethyl acetamide or ethylene glycol monomethyl ether is not conventional. Usually the most common alternative to benzene is toluene, in so far as both the solvents are highly apolar, whilst this is not the case with the solvents of possible use in the process of the present invention.

In order to illustrate the invention better, the following examples are now given.

### EXAMPLE 1

### Synthesis of 1,2,5,6-tetrahydro-8-methyl-pyrazine[3,2,1-j,k]-4H-carbazol

6-methyl-1-(2-chloroethyl-imino)-1,2,3,4-tetrahydrocarbazol hydrochloride (29.7 g, 0.1 mol.) was dissolved in dimethyl formamide (59.4 g), then potassium carbonate (29.7 g, 0.21 mol.) and tetrabutylammonium bromide (0.9 g) were added. The reaction mixture was heated to 120-125°C for approximately 2 hours until the starting product disappeared (detection via TLC). The salts formed in the reaction mixture were eliminated by filtration, and the resultant solution was used as if was in the subsequent phase.

### EXAMPLE 2

### Synthesis of pirlindole hydrochloride

To the solution obtained in Example 1 sodium borohydride (3.8 g, 0.1 mol.) was added, at 25-30°C, and the temperature was allowed to rise up to 90-100°C, then kept at these values until the starting product disappeared. By addition of hydrochloric acid a crystallyzed formed, which was separated by filtration and cooled. The solid obtained was recrystallized from water/sec-butanol to yield 18.1 g of the compound in question (yield calculated on 6-methyl-1-(2-chloroethylimino)-1,2,3,4-tetrahydrocarbazol hydrochloride: 69.1%).
Purity >99.8% (HPLC).

## Claims

1. Process for the synthesis of pirlindole hydrochloride of formula (I) 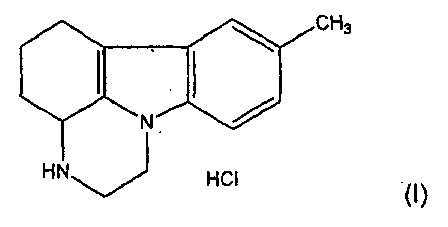 **characterized in that** the 6-methyl-1-(2-chloroethyl-imino)-1,2,3,4-tetrahydrocarbazol hydrochloride of formula (IV) 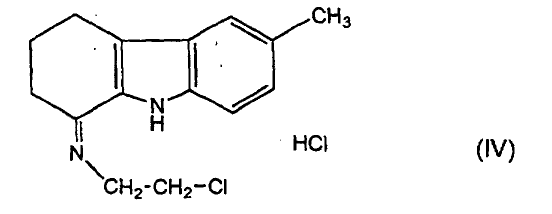 cyclizes with an alkaline agent, in the presence of a phase-transfer catalyst to yield the 1,2,5,6-tetrahydro-8-methyl-pyrazine[3,2,1-j,k]-4H-carbazol of formula (VI) 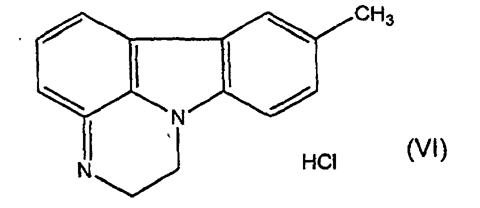 which is subjected to reduction.

2. Process according to Claim 1, in which the alkaline agent is present in a molar ratio of from 1.5:1 to 3:1 with respect to the compound of formula (IV) and the phase-transfer catalyst at 3 wt%.

3. Process according to Claim 2, in which the alkaline agent is an alkaline carbonate chosen from the group comprising potassium carbonate and sodium carbonate.

4. Process according to Claim 2, in which the alkaline agent is used in a ratio of 2.4:1.1 with respect to the compound of formula (IV).

5. Process according to Claim 1, in which the product of formula (VI) is reduced with sodium borohydride.

6. Process according to Claim 1, which is carried out in a solvent chosen from the group consisting of dimethyl formamide, dimethyl acetamide and ethylene glycol monomethyl ether.

7. Process according to Claim 6, in which the solvent is dimethyl formamide.

## Patentansprüche

1. Verfahren zur Synthese von Pirlindol-hydrochlorid der Formel (I): 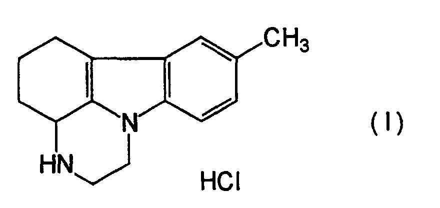 **dadurch gekennzeichnet, dass** 6-Methyl-1-(2-chlorethyl-imino)-1,2,3,4-tetrahydrocarbazolhydrochlorid der Formel (IV): 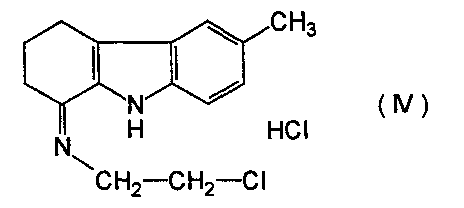 mit einem Alkali in Gegenwart eines Phasentransferkatalysators cyclisiert, zum Erhalt des 1,2,5,6-Tetrahydro-8-methyl-pyrazin[3,2,1-j,k]-4H-carbazols der Formel (VI): 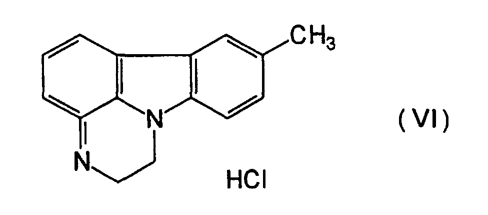 das einer Reduktion unterworfen wird.

2. Verfahren gemäss Anspruch 1, worin das Alkali in einem molaren Verhältnis von 1,5:1 bis 3:1, bezogen auf die Verbindung der Formel (IV), und der Phasentransferkatalysator zu 3 Gew.% anwesend ist.

3. Verfahren gemäss Anspruch 2, worin das Alkali ein Alkalicarbonat ist, ausgewählt aus der Gruppe, umfassend Kaliumcarbonat und Natriumcarbonat.

4. Verfahren gemäss Anspruch 2, worin das Alkali in einem Verhältnis von 2,4:1,1, bezogen auf die Verbindung der Formel (IV), verwendet wird.

5. Verfahren gemäss Anspruch 1, worin das Produkt der Formel (VI) mit Natriumborhydrid reduziert wird.

6. Verfahren gemäss Anspruch 1, das in einem Lösungsmittel, ausgewählt aus der Gruppe, bestehend aus Dimethylformamid, Dimethylacetamid und Ethylenglykolmonomethylether, durchgeführt wird.

7. Verfahren gemäss Anspruch 6, worin das Lösungsmittel Dimethylformamid ist.

## Revendications

1. Procédé de synthèse de chlorhydrate de pirlindole de formule (I): 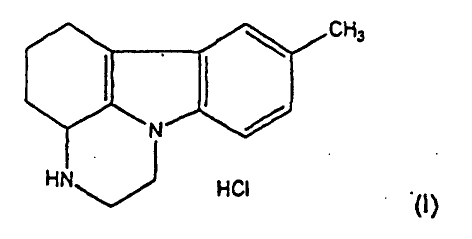 **caractérisé en ce que** l'on cyclise un chlorhydrate de 6-méthyl-1-(2-chloroéthyl-imino)-1,2,3,4-tétrahydrocarbazole de formule (IV) 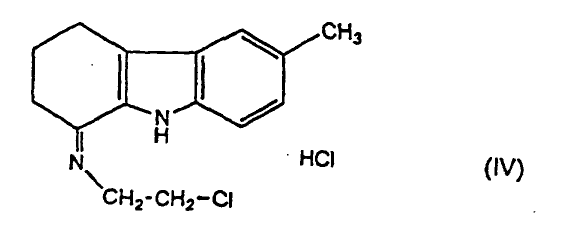 avec un agent alcalin en présence d'un catalyseur de transfert de phase pour produire le 1,2,5,6-tétrahydro-8-méthyl-pyrazine[3,2,1-j,k]-4H-carbazole de formule (VI) 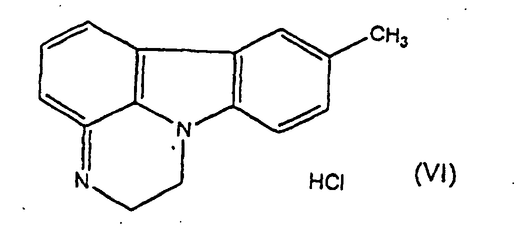 qui est soumis à une réduction.

2. Procédé selon la revendication 1, dans lequel l'agent alcalin est présent dans un rapport molaire de 1,5:1 à 3:1 par rapport au composé de formule (IV) et le catalyseur de transfert de phase à 3 % en poids.

3. Procédé selon la revendication 2, dans lequel l'agent alcalin est un carbonate alcalin choisi dans le groupe comprenant le carbonate de potassium et le carbonate de sodium.

4. Procédé selon la revendication 2, dans lequel l'agent alcalin est utilisé dans un rapport de 2,4:1,1 par rapport au composé de formule (IV).

5. Procédé selon la revendication 1, dans lequel le produit de formule (VI) est réduit par le borohydrure de sodium.

6. Procédé selon la revendication 1, qui est réalisé dans un solvant choisi dans le groupe constitué par le diméthylformamide, le diméthylacétamide, et l'éther mono-méthylique d'éthylène glycol.

7. Procédé selon la revendication 6, dans lequel le solvant est le diméthylformamide.
